(19) **Europäisches Patentamt**
**European Patent Office**
**Office européen des brevets**

(11) **EP 3 536 384 B1**

(12) **EUROPEAN PATENT SPECIFICATION**

(45) Date of publication and mention
of the grant of the patent:
**15.02.2023 Bulletin 2023/07**

(21) Application number: **19160969.2**

(22) Date of filing: **06.03.2019**

(51) International Patent Classification (IPC):
*A61Q 5/02* (2006.01)    *A61Q 5/12* (2006.01)
*A61K 8/73* (2006.01)    *A61Q 19/10* (2006.01)
*C11D 17/00* (1968.09)

(52) Cooperative Patent Classification (CPC):
**A61Q 5/02; A61K 8/60; A61K 8/608; A61K 8/73;**
**A61Q 5/12; A61Q 19/10**

(54) **COSMETIC COMPOSITION COMPRISING A CATIONIC INULIN AND AN ANIONIC SURFACTANT**

KOSMETISCHE ZUSAMMENSETZUNG MIT EINEM KATIONISCHEN DERIVAT AUS FRUCTAN
UND EINEM ANIONISCHEN ODER NICHTIONISCHEN TENSID

COMPOSITION COSMÉTIQUE COMPRENANT UN DÉRIVÉ CATIONIQUE DE FRUCTANE ET UN
TENSIOACTIF ANIONIQUE OU NON IONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.03.2018 EP 18160111**

(43) Date of publication of application:
**11.09.2019 Bulletin 2019/37**

(60) Divisional application:
**22211459.7**

(73) Proprietor: **Coöperatie Koninklijke Cosun U.A.**
**4814 NE Breda (NL)**

(72) Inventors:
• **Brooijmans, Tom Willem Louis**
**5175 CD Loon op Zand (NL)**
• **Raaijmakers, Henricus Wilhelmus Carolina**
**4708 NP Roosendaal (NL)**

• **van Kats, Carlos Maria**
**3523 XK Utrecht (NL)**

(74) Representative: **Nederlandsch Octrooibureau**
**P.O. Box 29720**
**2502 LS The Hague (NL)**

(56) References cited:
**FR-A1- 2 795 953    FR-A1- 2 795 954**

• **VAN ENGELEN G ET AL: "Clean products from a
clean planet for a clean future", INTERNET
CITATION, 1 October 2010 (2010-10-01), pages
58-62, XP002768951, Retrieved from the Internet:
URL:http://www.cosunbiobased.com/getmedia/
878020cd-160c-4f8e-858d-cc2d68b32ff6/SOFW_
October-2010_Clean-Products-from-a-Clean-P
lanet-for-a-Clean-Future.pdf?ext=.pdf [retrieved
on 2017-04-05]**

## Description

### Field of the invention

The invention relates to a cosmetic hair care composition comprising a cationic inulin and an anionic surfactant. The cosmetic hair care composition shows an improved capability of forming coacervates. The invention further relates to the use of the cationic inulin in combination with an anionic surfactant for repairing damaged hair.

### Background art

Cosmetic products such as hair care products generally comprise surfactants. Shampoos generally comprise anionic surfactants as they provide good cleaning power and good lathering. Although very good in removing sebum and dirt, anionic surfactants may cause an increase in electrical negative charges on the hair surface and increase frizz. Thoroughly cleansed hair may be difficult to comb, either wet or dry. Furthermore combing may cause damage to the hair structure or hair fibre, and may for example cause split ends and hair breakage.

Conditioners, either applied as aftershampoo or as conditioning shampoo, are used to decrease friction, to detangle the hair, to minimize frizz, to improve shine, to moisturize and/or to improve combability. Conditioners act by neutralizing the electrical negative charge of the hair fiber by adding positive charges and generally comprise cationic compounds such as quaternary ammonium compounds. Conditioners are generally applied as aftershampoo, i.e. applied in a separate stage after shampooing. The formulation of aftershampoos is rather easy but aftershampoos have the drawback that their use is inconvenient because of the necessity to apply the aftershampoo to the hair in a separate stage after the shampooing stage. Conditioning shampoo comprising an anionic surfactant as well as cationic compounds are convenient to use as they do not require an additional stage to apply. However as known by those skilled in the art the formulation of compositions comprising an anionic surfactant as well as cationic compounds is often challenging because of the inherent incompatibility between anionic surfactants and cationic compounds. Contact between an anionic surfactant and a cationic compound generally results in a precipitate.

FR2795953A1 discloses cosmetic compositions comprising cationic inulin and surfactants.

### Summary of the invention

It is an object of the present invention to provide a cosmetic composition such as a hair care product comprising both a cationic derivate of fructan and at least one anionic surfactant, non-ionic surfactant and/or amphoteric surfactant that is optically clear.

It is another object to provide a cosmetic composition, in particular a hair care composition, that has an improved deposition activity.

Furthermore, it is an object of the present invention to provide a cosmetic composition that comprises inherent biodegradable cationic derivates of fructan.

### Short description of drawings

The present invention will be discussed in more detail below, with reference to the attached drawings, in which

- Figure 1 shows the deposition profile (thickness and mass adsorbed) of a cationic derivate on hydrophilic silica as a function of surfactant concentration;
- Figure 2 and Figure 3 show the mass adsorbed of different cationic compounds to hydrophilic silica as a function of surfactant concentration;
- Figure 4 and Figure 5 show the mass adsorbed of cationic compounds according to the present invention and of some cationic compounds known in the art to hydrophobized silica as a function of surfactant concentration.

### Description of embodiments

A first aspect of the present invention relates to a cosmetic hair care composition comprising cationic inulin and at least one anionic surfactant, wherein the weight ratio of anionic surfactant to cationic inulin is more than 25:1, wherein the cationic inulin has a degree of substitution ranging between 0.6 and 3, and wherein the at least one anionic surfactant is selected from the group consisting of alkyl sulfates.

Possibly, the cosmetic hair care composition comprises the cationic inulin and an anionic surfactant in combination with a non-ionic surfactant or the cosmetic hair care composition comprises the cationic inulin and an anionic surfactant in combination with an amphoteric surfactant or the cosmetic hair care composition comprises the cationic inulin and an anionic surfactant in combination with a non-ionic surfactant and further in combination with an amphoteric surfactant.

The at least one anionic surfactant can be present as a monomer.

For the purpose of the present application "a cationic derivate of fructan" is understood to be a derivate of fructan comprising a cationic group. The cationic group may comprise an ammonium group, a quaternary ammonium group, a sulfonium group, a phosphonium group, a transitional metal or any other positively charged functional group. A preferred cationic group is a quaternary ammonium group. In highly preferred embodiments the cationic inulin is hydroxypropyltrimonium inulin.

For the purpose of this application "fructans" are understood to comprise all polysaccharides which have a multiplicity of anhydrofructose units. The fructans can have a polydisperse chain length distribution and can be straight-chain or branched. The fructans comprise both products obtained directly from a vegetable or other source and products in which the average chain length has been modified (increased or reduced) by fractionation, enzymatic synthesis or hydrolysis. The fructans have an average chain length (=degree of polymerisation, DP) of at least 2 to about 1000, in particular between 3 and 60, for example 3, 4, 5, 6, 7, 8, 15 or 25.

Surprisingly it has been found, that the cationic inulin has preferably an average molecular weight lower than 30000 g/mol and more preferably an average molecular weight ranging between 500 g/mol and 30000 g/mol. In preferred embodiments the average molecular weight of the cationic inulin ranges between 1000 g/mol and 15000 g/mol and more preferably between 2000 g/mol and 5000 g/mol.

Cationic compounds known in the art for use in cosmetic compositions generally have a molecular weight higher than 100 000 g/mol or even higher than 1 000 000 g/mol.

For the purpose of this application "average molecular weight" is understood to mean "weight average molecular weight" and is defined by the following formula :

$$Mw = \frac{\sum N_i M_i^2}{\sum N_i M_i}$$

With

$M_i$: the molecular weight of a chain
$N_i$: the number of chains of that molecular weight.

The average molecular weight may be calculated based on the average molecular weight of the cationic inulin, as determined by a chromatographic method such as HPAEC-PAD (high-performance anion exchange chromatography coupled to pulsed amperometric detection) before quaternization, and the weight increase based on the degree of substitution determined after quaternization.

The degree of substitution of the cationic inulin ranges preferably between 0.6 and 2, or between 0.6 and 1.3.

The "degree of substitution" is defined as the cationic group content per monosaccharide unit.

The solubility of the cationic inulin in water at a temperature of 25 °C is preferably higher than 20 wt%, for example higher than 30 wt%, higher than 40 wt%, higher than 45 wt%, higher than 50 wt%, higher than 60 wt% or higher than 70 wt%.

"Solubility" is defined as the maximum percentage (by weight) of a substance that will dissolve in a unit of volume of water at a certain temperature.

For the purpose of this application "inulins" are understood to comprise polysaccharides comprising β(2,1) linked fructofuranose units and a glucopyranose unit. The degree of polymerisation ranges preferably between 2 and 60. Inulin can for example be obtained from chicory, dahlias and Jerusalem artichokes.

For the purpose of the present application "a cationic derivate of inulin" is understood to be a derivate of inulin comprising a cationic group. The cationic group may comprise an ammonium group, a quaternary ammonium group, a sulfonium group, a phosphonium group, a transitional metal or any other positively charged functional group. A preferred cationic group is a quaternary ammonium group. In case the cationic group is a quaternary ammonium group, the degree of substitution may be determined based on the nitrogen content calculated using Kjeldahl method. Cationic inulin is known and sold under the trademark Quatin® (a trademark of Cosun Biobased Products).

The cationic inulin has preferably an average molecular weight of less than 30000 g/mol and more preferably an average molecular weight ranging between 500 g/mol and 30000 g/mol. In preferred embodiments the average molecular weight of the cationic inulin ranges between 1000 g/mol and 15000 g/mol and more preferably between 2000 g/mol and 5000 g/mol.

The cationic inulin preferably has a degree of substitution ranging between 0.6 and 1.3.

In embodiments, the cationic inulin has an average molecular weight in the range of 3000-5000 g/mol, preferably in the range of 3500-4500 g/mol, most preferably in the range of 3800-4200 g/mol.

In embodiments, the cationic inulin has:

- a degree of substitution in the range of 0.6 to 0.8, more preferably within the range of 0.65 to 0.75; and
- an average molecular weight in the range of 3000-5000 g/mol, preferably in the range of 3500-4500 g/mol, most preferably in the range of 3800-4200 g/mol.

In embodiments, the cationic inulin has a degree of substitution in the range of 1.15 to 1.45, preferably within the range of 1.2 to 1.4, more preferably within the range of 1.25 to 1.35.

In embodiments, the cationic inulin has an average molecular weight in the range of 4000-6000 g/mol, preferably in the range of 4500-5500 g/mol, most preferably in the range of 4800-5200 g/mol.

In embodiments, the cationic inulin has:

- a degree of substitution in the range of 1.15 to 1.45, preferably within the range of 1.2 to 1.4, more preferably within the range of 1.25 to 1.35; and
- an average molecular weight in the range of 4000-6000 g/mol, preferably in the range of 4500-5500 g/mol, most preferably in the range of 4800-5200 g/mol.

In embodiments the cationic inulin has an average molecular weight in the range of 2000-4000 g/mol, preferably in the range of 2500-3500 g/mol, most preferably in the range of 2800-3200 g/mol.

The cationic inulin has preferably a solubility in water at a temperature of 25 °C higher than 20 wt%, for example higher than 30 wt%, higher than 40 wt%, higher than 45 wt%, higher than 50 wt%, higher than 60 wt%, higher than 70 wt% and higher than 80 wt%.

For the purpose of this application "anionic surfactant" is defined as a surfactant comprising at least one anionic functional group. Preferred anionic surfactants are surfactants whereby all ionic or all ionisable groups comprise anionic groups. Preferred anionic groups comprise a sulfate group, a sulfonate group, a carboxylate group, a phosphate group or any other negatively charged functional group.

Examples of anionic surfactants comprising a sulfate group comprises : alkyl sulfates (AS) such as ammonium lauryl sulfate and sodium lauryl sulfate (SLS); alkyl ether sulfates (AES) such as sodium laureth sulfate also known as sodium lauryl ether sulfate (SLES) and sodium myreth sulfate.

Examples of anionic surfactants comprising a sulfonate group comprise alkyl benzene sulfonate, in particular linear alkylbenzene sulfonates (LABs) such as sodium linear alkyl benzene sulfonate; alkyl ester sulfonate, such as methyl ester sulfonate (MES) or a alfa olefin sulfonate (AOS).

Examples of anionic surfactants comprising a carboxylate group comprise alkyl carboxylates such as sodium stearate, and sodium lauroyl sarcosinate.

Examples of anionic surfactants comprising a phosphate group comprise alkyl-aryl phosphates.

In all the examples mentioned alkyl refers to alkyl groups preferably comprising from 6 to 40 carbon atoms and more preferably comprising between 6 and 24 carbon atoms.

In embodiments, a cosmetic hair care composition comprising the cationic inulin and the at least one anionic surfactant is provided, wherein the composition does not comprise an alkyl ether sulfate.

In embodiments, a cosmetic hair care composition comprising the cationic inulin and the at least one anionic surfactant is provided, wherein the at least one anionic surfactant is the salt of a compound represented by R-X, wherein X represents a sulfate group and wherein R is selected from straight chain $C_5$-$C_{24}$ alkyl groups.

In preferred embodiments, the anionic surfactant is provided in the form of a salt, preferably in the form of an alkali metal salt (such as a sodium salt), an ammonium salt, an aminoalcohol salt or a magnesium salt.

In embodiments a cosmetic hair care composition comprising the cationic inulin and the at least one anionic surfactant as defined herein is provided, comprising more than 0.1 wt% anionic surfactant, such as more than 1 wt%, more than 2 wt%, more than 3 wt%, more than 5 wt%, more than 7 wt%, more than 9 wt%, more than 10 wt%, more than 12 wt%, or more than 15 wt.% anionic surfactant.

In embodiments a cosmetic hair care composition comprising the cationic inulin and the at least one anionic surfactant is provided, comprising less than 30 wt% anionic surfactant, such as less than 25 wt%, less than 20 wt%, less than 15 wt%, less than 10 wt%, less than 5 wt%, or less than 4 wt% anionic surfactant.

In preferred embodiments a cosmetic hair care composition comprising the cationic inulin and the at least one anionic surfactant as defined herein is provided, wherein the weight ratio of anionic surfactant to cationic inulin is more than 30:1 or more than 40:1. In embodiments the weight ratio of the anionic surfactant to cationic inulin is more than 200:1, preferably more than 400:1, more than 600:1, or more than 1000:1.

In embodiments a cosmetic hair care composition comprising the cationic inulin and the at least one anionic surfactant as defined herein is provided, wherein the weight ratio of anionic surfactant to cationic inulin is more than 25:1, more than 30:1 or more than 40:1, and the concentration of the cationic inulin is in the range of 0.005-0.015 wt.%.

In embodiments a cosmetic hair care composition comprising the cationic inulin and the at least one anionic surfactant as defined herein is provided, wherein the weight ratio of anionic surfactant to cationic inulin is more than 25:1, more than 30:1 or more than 40:1, and the concentration of the cationic inulin is in the range of 0.1-1 wt.%.

The maximum weight ratio of anionic surfactant to cationic inulin is not particularly limited, although out of practical and/or economic considerations, a cosmetic hair care composition comprising a cationic inulin and the at least one anionic surfactant is provided, wherein the weight ratio of the anionic surfactant to cationic inulin is less than 1000:1, preferably less than 500:1, or less than 100:1.

In embodiments a cosmetic hair care composition comprising the cationic inulin and the at least one anionic surfactant as defined herein is provided, wherein the weight ratio of anionic surfactant to cationic inulin is in the range of 30:1 to 70:1, in the range of 40:1 to 60:1, or in the range of 45:1 to 55:1.

For the purpose of this application "non-ionic surfactant" is defined as a surfactant not containing an ionic group. Examples of non-ionic surfactants comprise ethoxylates, alkoxylates and cocamides. Particularly preferred non-ionic surfactants comprise alkyl polyglycosides (APGs).

The cosmetic hair care composition according to the present invention may further comprise an amphoteric surfactant as for example selected from betaines.

The cosmetic hair care composition according to the present invention can be formulated in various forms. The cosmetic hair care composition is preferably in the form of a liquid composition, more preferably an aqueous composition or in the form of a paste.

A cosmetic hair care composition according to the present invention comprises preferably between 0.1 wt% and 1 wt% of the cationic inulin. More preferably, a cosmetic hair care composition according to the present invention comprises between 0.1 wt% and 0.8% or between 0.2 wt% and 0.6 wt% of the cationic inulin.

A cosmetic hair care composition according to the present invention comprises preferably more than 0.01 wt% of the cationic inulin, such as more than 0.05 wt%, more than 0.1 wt%, more than 0.2 wt%, more than 0.3 wt%, more than 0.5 wt%, more than 0.6 wt%, more than 0.7 wt%, more than 0.9 wt%, more than 1 wt%, more than 2 wt%, or more than 5 wt.% of the cationic inulin.

A cosmetic hair care composition according to the present invention comprises preferably less than 10 wt% of the cationic inulin, such as less than 9 wt%, less than 6 wt%, less than 5 wt%, less than 4 wt%, less than 2 wt%, less than 1 wt%, less than 0.9 wt%, less than 0.8 wt%, less than 0.6 wt%, less than 0.4 wt%, or less than 0.1 wt% of the cationic inulin.

The cosmetic hair care composition according to the present invention comprises for example between 0.1 wt% and 1 wt% of a cationic inulin. Preferred embodiments comprise between 0.1 wt% and 0.8 wt% or between 0.2 wt% and 0.6 wt% of a cationic inulin.

The cosmetic hair care composition according to the present invention comprises preferably shampoos, conditioners, after-shampoos, two-in-one products, hair coloring products, or hair lotions.

The cosmetic hair care composition according to the present invention comprises preferably shampoos, hair damage repairing products, hair color protecting products, conditioners, after-shampoos, hair coloring products or hair lotions.

The cosmetic hair care composition according to the present invention may further comprise additional ingredients such as additional surfactants, preservating agents, viscosity modifiers, sequestering agents, pH adjusting agents, foam boosters, fragrances, vitamins and provitamins, builders, polymers, solubilizers, antioxidants, anti-dandruff agents, anti-seborrhoeic agents, agents for preventing hair loss and/or for promoting hair (re)growth, and any other additive conventionally used in the cosmetic fields.

The additional ingredients may be present in the composition according to the present invention in an amount ranging from about 0 to 5 wt%, relative to the total weight of the composition.

In preferred embodiments, the cosmetic hair care composition in accordance with the invention comprises the cationic inulin, the anionic surfactant as described herein and more than 50 wt.% of water, preferably more than 60 wt.%, more than 70 wt.% or more than 80 wt.% of water.

In embodiments, a cosmetic hair care composition is provided comprising the cationic inulin, the anionic surfactant as defined herein, more than 50 wt.% of water, preferably more than 60 wt.%, more than 70 wt.% or more than 80 wt.% of water, and less than 7 wt.%, preferably less than 5 wt.%, more preferably less than 4.5 wt.%, more preferably less than 3 wt.% of ingredients other than water, anionic surfactant and cationic inulin.

In embodiments, a cosmetic hair care composition is provided comprising the cationic inulin, the anionic surfactant as defined herein, more than 50 wt.% of water, preferably more than 60 wt.%, more than 70 wt.% or more than 80 wt.% of water, and less than 7 wt.%, preferably less than 5 wt.%, more preferably less than 4.5 wt.%, more preferably less than 3 wt.% of ingredients other than water, anionic surfactant and cationic inulin, wherein the at least one anionic surfactant is the salt of a compound represented by R-X, wherein X represents a sulfate group and wherein R is selected from straight chain $C_5$-$C_{24}$ alkyl groups.

In embodiments the cosmetic hair care composition in accordance with the invention does not comprise any other conditioning agent, preferably the cosmetic hair care composition does not comprise another conditioning agent selected from the group consisting of synthetic oils, mineral oils, vegetable oils, fluorinated or perfluorinated oils, natural or synthetic waxes, silicones, cationic polymers, fatty amines, fatty acids and derivatives thereof and fatty alcohols and derivatives thereof.

The cosmetic hair care composition according to the present invention shows an improved capability of forming a coacervate. The term "coacervate" refers to an insoluble complex formed between the cationic polymer and the surfactant(s) when diluted with water. A coacervate contains a high level of cationic charge and is known to deposit the polymer on hair and enhance the adsorption of insoluble actives.

The cationic inulin has the advantage that it is easy to process. For the preparation of a cosmetic hair care composition according to the present invention no additional handling is required to create hydration. Compared to compositions known in the art, the preparation according to the present invention does not require high shear treatment and does not require high temperature nor pH adjustments.

As shown in the appended examples, the cosmetic hair care compositions of the present invention may advantageously be provided in transparent form, even at concentrations where deposition occurs. Thus, in embodiments the cosmetic hair care compositions described herein are transparent or translucent. As used herein, transparency or translucency refers transparency or translucency in the visible spectrum of light. In embodiments the cosmetic hair care compositions described herein are characterized by a total transmittance of visible light (380-780 nm) of more than 80%, preferably more than 90%, more preferably more than 95% when determined over a path length of 1 cm.

In embodiments the cosmetic hair care compositions described herein have a turbidity of less than 100 FNU (Formazin Nephelometric Units), preferably less than 50 FNU, most preferably less than 10 FNU.

In preferred embodiments the cationic inulin and anionic surfactant are present at concentrations where deposition occurs on a hydrophilic silica surface, as determined using a Rudolph thin film ellipsometer, type 436 (Rudolph Research, Fairfield, NY), equipped with a xenon arc lamp and high-precision step motors, controlled by a personal computer, measured at a wavelength of 4015 Å and an angle of incidence of 67.87°. In preferred embodiments the cationic inulin and anionic surfactant are present at concentrations where deposition occurs at a hydrophilic surface, as determined using a Rudolph thin film ellipsometer, type 436 (Rudolph Research, Fairfield, NY), equipped with a xenon arc lamp and high-precision step motors, controlled by a personal computer, measured at a wavelength of 4015 Å and an angle of incidence of 67.87° and no macroscopic phase separation can be visually observed.

A method of treating hair is described wherein the cosmetic hair care composition is applied on the hair. The composition can be rinsed out the hair after a certain period of time, for example after a few minutes. Alternatively, for some compositions it is preferred to leave the composition on the hair, without further rinsing.

A second aspect of the invention, concerns the use of a cationic inulin in combination with an anionic surfactant, wherein the cationic inulin has a degree of substitution ranging between 0.6 and 3, wherein the at least one anionic surfactant is selected from the group consisting of alkyl sulfates and wherein the weight ratio of anionic surfactant to cationic inulin is more than 25:1, for repairing damaged hair. In preferred embodiments the anionic surfactant is sodium dodecyl sulfate.

The invention will now be described by comparing the deposition profile of a number of cationic compounds. Furthermore the influence of the cationic compounds on the combing force is evaluated.

In a first series of tests the conditioning performance of a composition according to the present invention is evaluated. For such evaluation the ability of three cationic compounds according to the present invention to form coacervates and to deposit on model substrates (hydrophilic, negatively charged silica and hydrophobized silica modified with octysilane) is evaluated using ellipsometry. The deposition of the three compounds according to the present invention is compared with two cationic compounds that are commercially available and commonly used for hair products, namely cationic modified cellulose (UCare®, obtained from Amerchol) and cationic modified guar (NHance®, obtained from Ashland). According to the ellipsometry tests - described in detail below- first the adsorption of pure polymer is measured. Anionic surfactant (SDS) is then added stepwise directly in the cuvette in the instrument while the absorption is measured. Once the surfactant reaches a critical value, the formation of coacervates starts and the adsorption to the surface dramatically increases. At higher concentration the surplus of surfactant will result in re-solubilization of the coacervates and desorption from the surface. Consequently, the coacervate adsorption/desoption "profile" as a function of the surfactant concentration indicates the propensity of the polymer to form coacervates and thus gives information to compare various cationic compounds.

**Description of the ellipsometry tests**

Ellipsometry is an optical method that measures the changes in polarization of light upon reflection at a planar surface. The instrument used was a Rudolph thin film ellipsometer, type 436 (Rudaloph Research, Fairfield, NY), equipped with a xenon arc lamp and high-precision step motors, controlled by a personal computer. Measurements were performed at a wavelength of 4015 Å and an angle of incidence of 67.87°.

A 4-zone measurement was first performed in air and in liquid, to determine the complex refractive index (N = n - ik) of the substrate bulk material as well as the refractive index ($n_x$) and thickness ($d_x$) of the outermost oxide layer. Samples were then injected into the cuvette and the ellipsometric angles $\psi$ (psi) and $\Delta$ (delta) were recorded in situ. If the optical properties of the substrate and the ambient media are known, the mean optical thickness (df) and refractive index ($n_f$) of the growing film can be obtained numerically from the change in the optical angles $\psi$ and $\Delta$. The thickness and the refractive index were then used to calculate the adsorbed amount, $\Gamma$ (mg/m²), according to the de Feijter formula :

$$\Gamma = \frac{d_f(n_f - n_0)}{\frac{dn}{dc}}$$

With

$\Gamma$      the mass per surface area;
$d_f$      the thickness (mean thickness) of the adsorbed film;
$n_f$      the refractive index of the adsorbed film;
$n_0$      the refractive index of the bulk solution; and
dn/dc      the refractive index increment as a function of the bulk concentration.

This equation is based on the approximation that the increments in refraction increase linearly with concentration up to the concentration found at the surface. The dn/dc value was estimated to be 0.15 for the polymer surfactant complex.

The measurement cell system, where the substrate surface is emerged vertically in a 5 mL thermostated quartz cuvette, is a non-continuous flow system with continuous stirring, giving the possibility to rinse the cuvette solution between additions.

**Model surfaces**

Two types of model surfaces were used: hydrophilic, negatively charged silica, a model for damaged hair, and hydrophobized silica modified with octysilane (hydrophobic, C8), a model for virgin hair. The silica substrates were cleaned by boiling in acidic and basic solution and then plasma cleaned just prior to use, and the hydrophobic C8 surfaces were made by reacting the silanol groups on a clean silica substrate with dimethyl octylchlorosilane in gas phase in an evacuated desiccator overnight. The C8 surfaces were then sonicated three time for 5 minutes in trichloroethylene and three times for 5 minutes in ethanol, and finally rinsed with ethanol and MilliQ water prior to use. The water contact angle after modification was close to 90°, indicating an extremely hydrophobic surface, compared to less than 10° for hydrophilic silica.

**Materials**

The materials used are

- Sodium dodecyl sulfate (SDS) from Sigma Aldrich;
- Sodium chloride (NaCl) from Sigma Aldrich;
- Quatin® samples from Cosun;
- UCare® L400 from Amerchol;
- NHance® 3196 from Ashland;

All samples were used without further purification. The NHance® powder first needs to be suspended in MilliQ water, and then acidified with acetic acid to get into solution, before diluting it to the desired concentration for the stock solution. A summary of the cationic compounds is given in Table 1.

**Table 1**

| Sample | Compound | Degree of substitution | Charge density | Nitrogen content (range) [wt%] |
|--------|----------|------------------------|----------------|-------------------------------|
| 1 | UCare® LR400 | 0.15 | 0.71 | 0.91 (0.8-1.1) |
| 2 | NHance® 3196 | | | 1.28 (1.25-1.55) |

(continued)

| Sample | Compound | Degree of substitution | Charge density | Nitrogen content (range) [wt%] |
|---|---|---|---|---|
| 3 | Quatin® 350 | 0.37 | 1.5 | 1.34 |
| 4 | Quatin® 680 | 0.72 | 2.92 | 1.73 |
| 5 | Quatin® 1280 | 1.29 | 5.49 | 2.09 |

**Experimental set-up**

Cationic compound and surfactant (SDS) stock solutions were prepared in a 1 mM NaCl solution. In the experiments the cuvette was first filled with 4.5 mL of the pure 1 mM NaCl medium for baseline measurement. 0.5 mL of a 1000 ppm cationic compound solution was then added to the cuvette, yielding a final cationic compound concentration in the cuvette of 100 ppm, and the adsorption of the cationic compound to the clean substrate was monitored in-situ. Known small amounts of 1, 10, 100, or 1000 mM SDS Solutions were then progressively added to obtain the desired surfactant concentrations. The adsorption after each addition was allowed to reach a steady state, which took approximately 100-3000 s. Macroscopic phase separation was visually observed in the cuvette in the ellipsometer for most measurements, but was also separately assessed by shining a laser through glass vials at corresponding polymer surfactant mixtures to visually determine the light scattering.

**Results and Discussion**

Without being bound to any theory, it is accepted that the negatively charged surfactants associate to the cationic compound so that at the critical association concentration (CAC) a neutral complex (coacervate) is formed, leading to macroscopic phase separation. This macroscopic phase separation can be observed as a dramatic increase in turbidity of the bulk. The solubility of the complexes that are formed, is in general low, which leads to surface deposition, evident as an increased adsorption in ellipsometry measurements. The surface deposition in particular on hydrophilic damaged hair is an indication of the caring/repairing effect of the formulation of the hair product. Furthermore, as the cation surfactant complex is able to associate to silicon oil droplets present in the formulation, the formulation of cation surfactant complex may lead to increased oil deposition on the surface, by essentially bridging the surface of the oil droplet and the hair.

If the surfactant concentration is increased beyond the CAC, excess surfactant may associate to the cationic compound. The ability of a given cationic compound to associate with additional surfactant molecules is closely related to its hydrophobicity. Hydrophobic cationic compounds (or less hydrophilic) will associate more effectively to the tails of surfactants and therefore "bind" more surfactants. The surplus of negatively charged surfactants will lead to a net negative charge of the complex, which in turn will lead to increased solubility in the bulk as well as swelling at the surface. If the cationic compound is hydrophobic enough a second association concentration will be reached (CAC2). At this concentration (and above this concentration) the complex has a high negative charge and is re-solubilized, and therefore also easily detached from the surface.

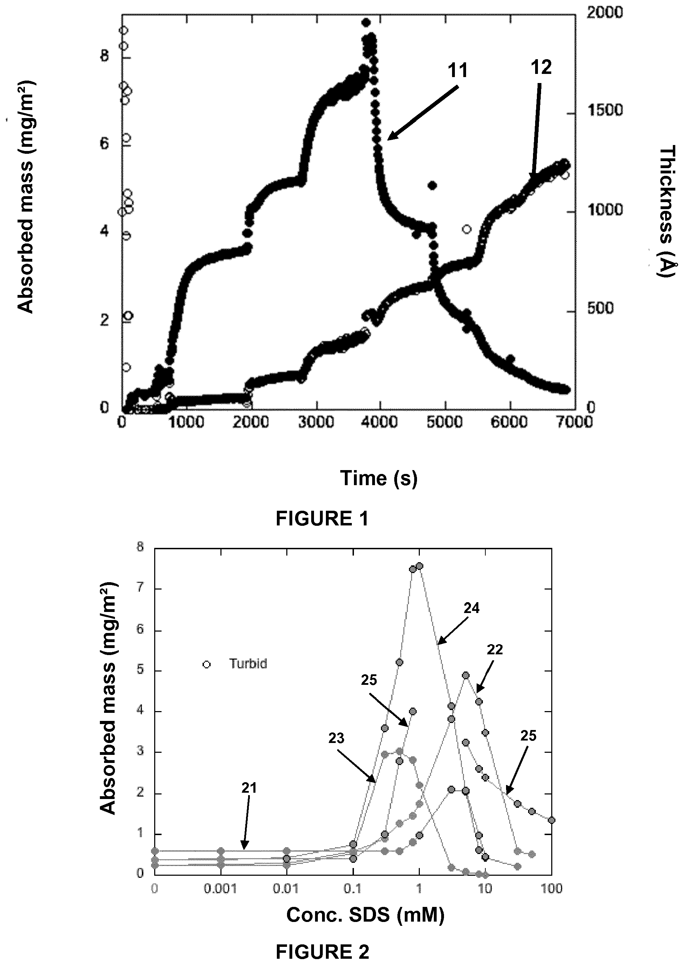

In the ellipsometry experiments, the cationic compound polymer was first added (without any surfactant present) and the deposition of the pure polyelectrolyte is measured. When surfactants are progressively added to reach higher and higher surfactant concentrations, the changes in surface deposition is evident as a change in adsorbed mass and its film thickness. Figure 1 shows the deposition profile (adsorbed mass as well as thickness) of Quatin® 680 as a function of time with increasing SDS concentration. Line 11 shows the thickness of the Quatin® 680/SDS complex to a hydrophilic silica substrate as a function of time during progressive additions of SDS. Line 12 shows the mass adsorbed of the Quatin® 680/SDS complex as a function of time during progressive additions of SDS. From the deposition profile shown in Figure 1, it can be derived that the mass and film thickness are low for the pure cationic compound, but with increasing SDS concentration the adsorbed mass increases drastically and the layer swells gradually. This continues until a maximum adsorbed mass is reached. Upon further increase of the SDS concentration, desorption and further swelling are observed.

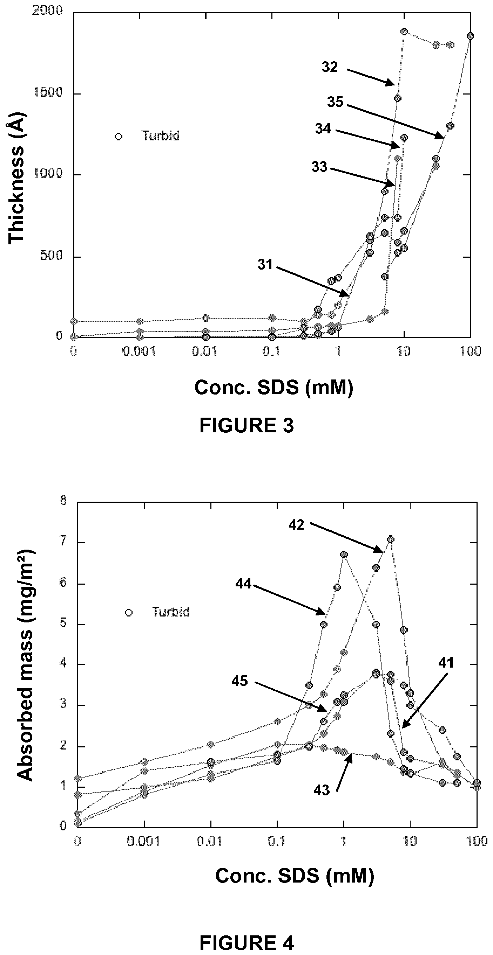

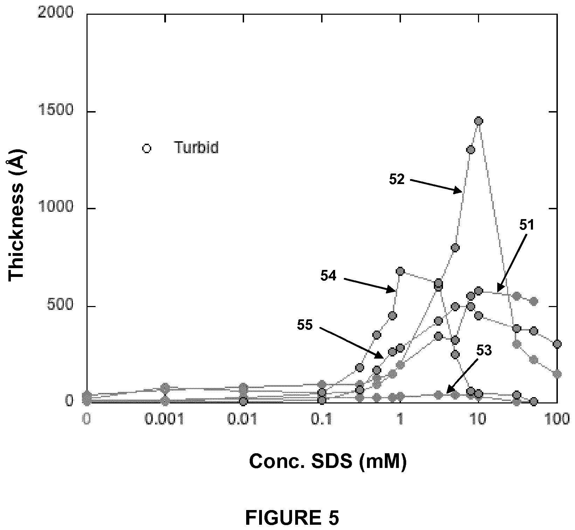

The adsorbed mass as a function of SDS concentration on hydrophilic silica for the different tested cationic compounds is given in Figure 2. Curve 21 shows the adsorbed mass of UCare®, curve 22 shows the adsorbed mass of NHance®, curve 23 shows the adsorbed mass of Quatin® 350, curve 24 shows the adsorbed mass of Quatin® 680 and curve 25 shows the adsorbed mass of Quatin® 1280. The layer thickness as a function of SDS concentration on hydrophilic silica is given in Figure 3. Curve 31 shows the layer thickness of UCare®, curve 22 shows the layer thickness of NHance®, curve 23 shows the layer thickness of Quatin® 350, curve 24 shows the layer thickness of Quatin® 680 and curve 25 shows the layer thickness of Quatin® 1280. The corresponding results obtained for the different cationic compounds for the adsorbed mass and layer thickness for deposition to hydrophobic substrates are given respectively in Figure 4 and Figure 5. Curve 41 shows the adsorbed mass of UCare®, curve 42 shows the adsorbed mass of NHance®, curve 43 shows the adsorbed mass of Quatin® 350, curve 44 shows the adsorbed mass of Quatin® 680 and curve 45 shows the adsorbed mass of Quatin® 1280. Curve 51 shows the layer thickness of UCare®, curve 52 shows the layer thickness of NHance®, curve 53 shows the layer thickness of Quatin® 350, curve 54 shows the layer thickness of Quatin® 680 and curve 55 shows the layer thickness of Quatin® 1280.

As can be seen, the critical association concentration (CAC) is lower for all Quatin® compounds compared to the other compounds tested. This means that the increased deposition as well as the maximum deposition is for all Quatin® compounds reached at lower SDS concentration compared to UCare® and NHance®. As can be derived from the Figures 2-5, the maximum deposition is reached at lower SDS concentrations for Quatin® 350, slightly higher for Quatin® 680, and even higher for Quatin® 1280. This means that the maximum deposition follows the degree of substitution of the polymers. A higher positive charge translates as a larger amount of SDS being required to reach charge-neutrality.

As mentioned earlier, the second association concentration (CAC2) (the SDS concentration where the complex gets overcharged and is re-dissolved) is more related to the hydrophobicity of the polymer. A hydrophobic (or less hydrophilic) polymer (such as UCare®) associates more with the hydrophobic tails of SDS, and can thus more easily be overcharged. NHance® on the other hand is more hydrophilic and thus needs higher concentrations of SDS to obtain overcharging.

The results for the Quatin® samples indicate that the SDS complexes with Quatin® 1280, and Quatin® 680 is never completely re-dissolved (since they are turbid at all high SDS concentrations).

Quatin® 1280 was even too turbid for ellipsometry measurements at the deposition maxima. A high CAC2 value means that the shampoo does not need to be diluted that much in the shower for deposition to occur which should be beneficial. Furthermore, a wide range from CAC to CAC2 would imply that the deposition can occur at many different ratios and therefore may be less sensitive during rinsing in the shower.

Quatin® 350 is of special interest since it does not lead to phase separate at all (no turbidity was seen in the ellipsometer cuvette). For Quatin® 350 no scattering was observed by visual inspection when shining a laser through the sample. It is also interesting that Quatin® 350 complexes still deposit to hydrophilic surfaces, whereas the deposition is minimal to hydrophobic surfaces. The hydrophilic surface is a model for damaged hair, which is the situation where you want deposition to occur, while the hydrophobic surface would model virgin hair and is therefore in less need for deposition.

In a second series of tests the reduction in wet combing force between untreated tresses and tresses treated with a cationic fructan in water is evaluated. Three different cationic compounds (0.4 wt%) in water are compared. The test samples are referred to as sample A, sample B and sample C. The cationic compounds of Samples A to C are specified in Table 2. More details about the cationic compounds of Samples A to C are given in Table 3.

**Table 2** : Cationic compound for Sample A-C

| Sample | Additive | INCI name |
|--------|----------|-----------|
| Sample A | Quatin® 680 TQ-D | Hydroxypropyltrimonium inulin |
| Sample B | Quatin® 1280 TQ-D | Hydroxypropyltrimonium inulin |
| Sample C | Quatin® 350 TQ-D, not according to the invention | Hydroxypropyltrimonium inulin |

**Table 3** : Details of the cationic compounds used in Samples A-C

| Additive | Degree of substitution | Charge density | Molecular weight (g/mol) | Solubility in water | Viscosity 1 % @ 25°C (cps) | Transmittance (600 nm) |
|----------|------------------------|----------------|--------------------------|---------------------|-----------------------------|-------------------------|
| Quatin® 680 TQ-D | 0.68 | 2.92 | ± 4000 | High | 1,1 | 100 % |
| Quatin®1280 TQ-D | 1.28 | 5.49 | ± 5000 | High | 1,1 | 100 % |
| Quatin® 350 TQ-D, not according to the invention | 0.35 | 1.50 | ± 3000 | High | 1,1 | 100 % |

The wet combing force and the reduction in wet combing force is determined using the test procedure as described below.

**Description of the test procedure to determine the wet combining force**

**Hair tresses** :

The hair tresses used in the tests were European natural human hair bleached in a standardized procedure (500-700 mN wet combing force), 2 g of free hair, 21 cm length. For each test sample 5 tresses were used.

**Climatic conditions**

All investigations took place in an air-conditioned room at a temperature of 21 $\pm$ 1 °C and at 50 $\pm$ 5 % relative humidity.

**Standard washing procedure**

The tresses were wetted for at least 60 seconds in tap water (pH 7). Per gram hair, an amount of 0.2 ml standard shampoo was massaged into the hair for one minute. The shampoo was left on the hair for 30 seconds. The tresses were then rinsed for 90 seconds under running lukewarm tap water. Overnight, the hair tresses were acclimatized in an air-conditioned room at the above-mentioned climatic conditions.

**Sample preparation**

For samples A to C a composition with 0.2 wt% of active substances was obtained by adding 0.250 ml to 50 ml distilled water.

**Application mode**

Samples A to C were applied to the tresses by merging the tresses and the solution for 5 minutes.

**Test procedure**

First the tresses were weighed and the moist mass at 60 % moisture content was calculated after a conditioning phase overnight at the mentioned climatic conditions.
The hair tresses were washed using a standard shampoo and subsequently adjusted to the calculated moist mass.
After combing the tresses by hand, the baseline measurements were carried out ten times for each tress using a universal test machine (Zwicke Z0.5 TN, Zwick Ulm equipped with a load cell having a nominal force of up to 10 N and fine combing segment, into which the tresses were placed and then automatically combed. The universal test machine measured the force needed to comb the tresses.
Afterwards, the tresses were treated with the samples A to C as described above (see application mode).
Directly after application of the samples A to C, each tress was combed, adjusted to moist mass and measured ten time, using the universal test machine. For each sample, a clean comb was used. For the measurements, one combing segment was used through the whole test, and it was cleaned when switching to tresses treated with another sample.

**Analysis of the Data**

For each tress the arithmetic mean of all possible combinations was calculated using the following formula :

$$\frac{Combing\ force\ treated}{Combing\ force\ untreated} \qquad \text{(formula I).}$$

Having ten measured values for each tress (untreated and treated), this way of evaluation resulted in the arithmetical mean of 100 single quotients.
The reduction of the combing force was then calculated per tress according to the following formula:

$$RCF[\%]=(1- \text{Arithmetic Mean of Quotients according to formula I})*100 \qquad (\text{formula II})$$

## Statistical analysis

To describe the data, the mean and median with standard deviation of the original data and of the reduced combing force were computed. For statistical analysis a significance level of $\alpha=0.05$ was chosen. The data of the combing force usually follow a normal distribution.

A statistical comparison of the reduced combing force of each product to the benchmark of 0% was carried out by a two-sided one-sample t-test.

A comparison between the test products was done according to the following procedure: The combing force after treatment was analysed by an ANCOVA basing on mean values of each tress with covariate "combing force before treatment" (F-Test, post hoc analysis adjusted for multiplicity by Sidak).

Computation of the statistical data was carried out with a commercially available statistics program (SAS).

## The results

Mean values of combing force and reduced combing force of the test products as well as standard deviations, and median are presented in Table 4.

**Table 4** : Combining force before and after treatment and reduced combing force

| Sample | N | | Combing force before treatment ([mN]) | Combing force after treatment ([mN]) | Reduced Combing force [%] |
|---|---|---|---|---|---|
| A | 5 | Mean | **694.43** | **524.77** | **23.63** |
| | | Std. Dev. | 7.44 | 9.48 | 1.58 |
| | | Median | 692.91 | 525.46 | 24.40 |
| B | 5 | Mean | **685.93** | **568.73** | **16.18** |
| | | Std. Dev. | 5.59 | 20.76 | 3.15 |
| | | Median | 687.07 | 567.29 | 16.25 |
| C | 5 | Mean | **682.53** | **451.13** | **33.13** |
| | | Std. Dev. | 9.71 | 13.52 | 2.06 |
| | | Median | 684.59 | 455.99 | 33.24 |

For all sample the combing force decreased after treatment. The reduced combing force of the samples ranges between 16.2 % and 33.1 %.

The statistical comparison of the treatments with a benchmark of 0 % showed a significant ($p \leq 0.05$) difference for all tested samples.

## Claims

1. A cosmetic hair care composition comprising cationic inulin and at least one anionic surfactant, wherein the weight ratio of anionic surfactant to cationic inulin is more than 25:1, wherein the cationic inulin has a degree of substitution ranging between 0.6 and 3, and wherein the at least one anionic surfactant is selected from the group consisting of alkyl sulfates.

2. The cosmetic hair care composition according to claim 1 wherein the cationic inulin has a degree of substitution ranging between 0.6 and 1.3.

3. The cosmetic hair care composition according to any one of the preceding claims, wherein the cationic inulin has a solubility in water at a temperature of 25 °C of at least 20 wt%.

4. The cosmetic hair care composition according to any one of the preceding claims, wherein the cationic inulin comprises a quaternary ammonium group.

**5.** The cosmetic hair care composition according to any one of the preceding claims wherein the cationic inulin has:

> • a degree of substitution in the range of 0.6 to 0.8, preferably within the range of 0.65 to 0.75; and
> • an average molecular weight in the range of 3000-5000 g/mol, preferably in the range of 3500-4500 g/mol, most preferably in the range of 3800-4200 g/mol.

**6.** The cosmetic hair care composition according to any one of the preceding claims, wherein the composition does not comprise an alkyl ether sulfate.

**7.** The cosmetic hair care composition according to any one of the preceding claims, wherein the at least one anionic surfactant is the salt of a compound represented by R-X, wherein X represents a sulfate group and wherein R is selected from straight chain $C_5$-$C_{24}$ alkyl groups.

**8.** The cosmetic composition according to any one of the preceding claims, wherein the at least one anionic surfactant is sodium lauryl sulfate.

**9.** The cosmetic hair care composition according to any one of the preceding claims comprising more than 50 wt.% of water, preferably more than 60 wt.%, more than 70 wt.% or more than 80wt.% of water, and less than 7 wt.%, preferably less than 5 wt.%, more preferably less than 4.5 wt.%, more preferably less than 3 wt.% of ingredients other than water, the anionic surfactant and the cationic inulin.

**10.** The cosmetic hair care composition according to any one of the preceding claims which is transparent or translucent.

**11.** The cosmetic hair care composition according to any one of the preceding claims comprising 0.1-1 wt.% of the cationic inulin.

**12.** The cosmetic hair care composition according to any one of the preceding claims, wherein said cosmetic composition comprises a shampoo, hair damage repairing product, hair color protecting product, conditioner, after-shampoo, hair coloring product or hair lotion soap.

**13.** Use of a cationic inulin in combination with an anionic surfactant, wherein the cationic inulin has a degree of substitution ranging between 0.6 and 3, wherein the at least one anionic surfactant is selected from the group consisting of alkyl sulfates and wherein the weight ratio of anionic surfactant to cationic inulin is more than 25:1, for repairing damaged hair.


**Patentansprüche**

**1.** Kosmetische Haarpflegezusammensetzung, umfassend kationisches Inulin und mindestens ein anionisches Tensid, wobei das Gewichtsverhältnis von anionischem Tensid zu kationischem Inulin mehr als 25:1 beträgt, wobei das kationische Inulin einen Substitutionsgrad im Bereich zwischen 0,6 und 3 aufweist und wobei das mindestens eine anionische Tensid ausgewählt ist aus der Gruppe bestehend aus Alkylsulfaten.

**2.** Kosmetische Haarpflegezusammensetzung nach Anspruch 1, wobei das kationische Inulin einen Substitutionsgrad im Bereich zwischen 0,6 und 1,3 aufweist.

**3.** Kosmetische Haarpflegezusammensetzung nach einem der vorhergehenden Ansprüche, wobei das kationische Inulin eine Löslichkeit in Wasser bei einer Temperatur von 25 °C von mindestens 20 Gew.-% aufweist.

**4.** Kosmetische Haarpflegezusammensetzung nach einem der vorangehenden Ansprüche, wobei das kationische Inulin eine quartäre Ammoniumgruppe umfasst.

**5.** Kosmetische Haarpflegezusammensetzung nach einem der vorangehenden Ansprüche, wobei das kationische Inulin aufweist:

> • einen Substitutionsgrad im Bereich von 0,6 bis 0,8, bevorzugt im Bereich von 0,65 bis 0,75; und
> • ein durchschnittliches Molekulargewicht im Bereich von 3000-5000 g/mol, bevorzugt im Bereich von 3500-4500 g/mol, am meisten bevorzugt im Bereich von 3800-4200 g/mol.

6.  Kosmetische Haarpflegezusammensetzung nach einem der vorhergehenden Ansprüche, wobei die Zusammensetzung kein Alkylethersulfat enthält.

7.  Kosmetische Haarpflegezusammensetzung nach einem der vorhergehenden Ansprüche, wobei das mindestens eine anionische Tensid das Salz einer durch R-X dargestellten Verbindung ist; wobei X eine Sulfatgruppe darstellt und wobei R ausgewählt ist aus geradkettigen $C_5$-$C_{24}$-Alkylgruppen.

8.  Kosmetische Haarpflegezusammensetzung nach einem der vorhergehenden Ansprüche, wobei das mindestens eine anionische Tensid Natriumlaurylsulfat ist.

9.  Kosmetische Haarpflegezusammensetzung nach einem der vorhergehenden Ansprüche, umfassend mehr als 50 Gew.-% Wasser, bevorzugt mehr als 60 Gew.-%, mehr als 70 Gew.-% oder mehr als 80 Gew.-% Wasser, und weniger als 7 Gew.-%, bevorzugt weniger als 5 Gew.-%, noch bevorzugter weniger als 4,5 Gew.-%, noch bevorzugter weniger als 3 Gew.-% an anderen Bestandteilen als Wasser, dem anionischen Tensid und dem kationischen Inulin.

10. Die kosmetische Haarpflegezusammensetzung nach einem der vorhergehenden Ansprüche, die transparent oder durchscheinend ist.

11. Kosmetische Haarpflegezusammensetzung nach einem der vorangehenden Ansprüche, die 0,1-1 Gew.-% des kationischen Inulins enthält.

12. Kosmetische Haarpflegezusammensetzung nach einem der vorhergehenden Ansprüche, wobei die kosmetische Zusammensetzung ein Shampoo, ein Produkt zur Reparatur von Haarschäden, ein Produkt zum Schutz der Haarfarbe, eine Pflegespülung, ein After-Shampoo, ein Haarfärbeprodukt oder eine Haarwaschseife umfasst.

13. Verwendung eines kationischen Inulins in Kombination mit einem anionischen Tensid, wobei das kationische Inulin einen Substitutionsgrad im Bereich zwischen 0,6 und 3 aufweist, wobei das mindestens eine anionische Tensid aus der Gruppe ausgewählt ist, die aus Alkylsulfaten besteht, und wobei das Gewichtsverhältnis von anionischem Tensid zu kationischem Inulin mehr als 25:1 beträgt, zur Reparatur von geschädigtem Haar.

**Revendications**

1.  - Composition cosmétique de soins capillaires comprenant de l'inuline cationique et au moins un tensio-actif anionique, dans laquelle le rapport en poids du tensio-actif anionique à l'inuline cationique est supérieur à 25:1, dans laquelle l'inuline cationique a un degré de substitution se situant dans la plage entre 0,6 et 3, et dans laquelle ledit au moins un tensio-actif anionique est choisi dans le groupe consistant en alkyl sulfates.

2.  - Composition cosmétique de soins capillaires selon la revendication 1, dans laquelle l'inuline cationique a un degré de substitution se situant dans la plage entre 0,6 et 1,3.

3.  - Composition cosmétique de soins capillaires selon l'une quelconque des revendications précédentes, dans laquelle l'inuline cationique a une solubilité dans l'eau à une température de 25°C d'au moins 20 % en poids.

4.  - Composition cosmétique de soins capillaires selon l'une quelconque des revendications précédentes, dans laquelle l'inuline cationique comprend un groupe ammonium quaternaire.

5.  - Composition cosmétique de soins capillaires selon l'une quelconque des revendications précédentes, dans laquelle l'inuline cationique a :

    • un degré de substitution dans la plage de 0,6 à 0,8, de préférence dans la plage de 0,65 à 0,75 ; et
    • une masse moléculaire moyenne se situant dans la plage de 3000 à 5000 g/mol, de préférence dans la plage de 3500 à 4500 g/mol, de la façon que l'on préfère le plus dans la plage de 3800 à 4200 g/mol.

6.  - Composition cosmétique de soins capillaires selon l'une quelconque des revendications précédentes, dans laquelle la composition ne comprend pas d'alkyl éther sulfate.

7.  - Composition cosmétique de soins capillaires selon l'une quelconque des revendications précédentes, dans laquelle ledit au moins un tensio-actif anionique est le sel d'un composé représenté par R-X, où X représente un groupe sulfate et où R est choisi parmi les groupes alkyle en $C_5$-$C_{24}$ à chaîne droite.

8. - Composition cosmétique selon l'une quelconque des revendications précédentes, dans laquelle ledit au moins un tensio-actif anionique est le lauryl sulfate de sodium.

9. - Composition cosmétique de soins capillaires selon l'une quelconque des revendications précédentes comprenant plus de 50 % en poids d'eau, de préférence plus de 60 % en poids, plus de 70 % en poids ou plus de 80 % en poids d'eau, et moins de 7 % en poids, de préférence moins de 5 % en poids, de façon davantage préférée moins de 4,5 % en poids, de façon davantage préférée moins de 3 % en poids d'ingrédients autres que l'eau, le tensio-actif anionique et l'inuline cationique.

10. - Composition cosmétique de soins capillaires selon l'une quelconque des revendications précédentes qui est transparente ou translucide.

11. - Composition cosmétique de soins capillaires selon l'une quelconque des revendications précédentes comprenant 0,1 à 1 % en poids de l'inuline cationique.

12. - Composition cosmétique de soins capillaires selon l'une quelconque des revendications précédentes, dans laquelle ladite composition cosmétique comprend un shampooing, un produit de réparation des dommages capillaires, un produit de protection de la couleur des cheveux, un conditionneur, un après-shampooing, un produit de coloration des cheveux ou un savon lotion capillaire.

13. - Utilisation d'une inuline cationique en combinaison avec un tensio-actif anionique, dans laquelle l'inuline cationique a un degré de substitution se situant dans la plage entre 0,6 et 3, dans laquelle ledit au moins un tensio-actif anionique est choisi dans le groupe consistant en alkyl sulfates et dans laquelle le rapport en poids du tensio-actif anionique à l'inuline cationique est supérieur à 25:1, pour réparer des cheveux endommagés.

FIGURE 1

FIGURE 2

FIGURE 3

FIGURE 4

**FIGURE 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2795953 A1